(19) Europäisches Patentamt
European Patent Office
Office européen des brevets

(11) **EP 2 794 898 B1**

(12) **EUROPÄISCHE PATENTSCHRIFT**

(45) Veröffentlichungstag und Bekanntmachung des Hinweises auf die Patenterteilung:
**01.08.2018 Patentblatt 2018/31**

(21) Anmeldenummer: **12806038.1**

(22) Anmeldetag: **18.12.2012**

(51) Int Cl.:
*C12N 9/02* *(2006.01)*      *C12P 7/64* *(2006.01)*
*C12P 13/04* *(2006.01)*

(86) Internationale Anmeldenummer:
**PCT/EP2012/075889**

(87) Internationale Veröffentlichungsnummer:
**WO 2013/092547 (27.06.2013 Gazette 2013/26)**

(54) **VERFAHREN ZUR VERBESSERTEN ABTRENNUNG EINER HYDROPHOBEN ORGANISCHEN LÖSUNG VON EINEM WÄSSRIGEN KULTURMEDIUM**

METHOD FOR IMPROVED SEPARATION OF A HYDROPHOBIC ORGANIC SOLUTION FROM AN AQUEOUS CULTURE MEDIUM

PROCÉDÉ DE SÉPARATION AMÉLIORÉE D'UNE SOLUTION ORGANIQUE HYDROPHOBE ET D'UN MILIEU DE CULTURE AQUEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 EP 11195221**

(43) Veröffentlichungstag der Anmeldung:
**29.10.2014 Patentblatt 2014/44**

(73) Patentinhaber: **Evonik Degussa GmbH**
**45128 Essen (DE)**

(72) Erfinder:
• HENNEMANN, Hans-Georg
45770 Marl (DE)
• SCHAFFER, Steffen
45699 Herten (DE)
• WESSEL, Mirja
44799 Bochum (DE)
• HAAS, Thomas
48161 Münster (DE)
• CORTHALS, Jasmin
44879 Bochum (DE)
• BERSE, Katharina
45731 Waltrop (DE)
• HAFKEMEYER, Sabine
45770 Marl (DE)
• PÖTTER, Markus
201702 Shangai (CN)
• ERHARDT, Frank
33604 Bielefeld (DE)

(56) Entgegenhaltungen:
WO-A1-2009/077461      WO-A1-2010/021711

• YANGKAI DUAN ET AL: "De novo Biosynthesis of Biodiesel by Escherichia coli in Optimized Fed-Batch Cultivation", PLOS ONE, Bd. 6, Nr. 5, 23. Mai 2011 (2011-05-23), Seite E20265, XP055026594, DOI: 10.1371/journal.pone.0020265
• ERIC J. STEEN ET AL: "Microbial production of fatty-acid-derived fuels and chemicals from plant biomass", NATURE, Bd. 463, Nr. 7280, 28. Januar 2010 (2010-01-28), Seiten 559-562, XP055011271, ISSN: 0028-0836, DOI: 10.1038/nature08721

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Anmeldung betrifft ein Verfahren zur verbesserten Abtrennung einer hydrophoben organischen Lösung von einem wässrigen Kulturmedium umfassend die Schritte, Bereitstellen eines wässrigen Kulturmediums umfassend eine stoffwechselaktive Zelle, Kontaktieren des wässrigen Kulturmediums mit einer hydrophoben organischen Lösung, Abtrennen der hydrophoben organischen Lösung von dem wässrigen Kulturmedium, wobei die Zelle eine ihrem Wildtyp gegenüber verringerte Aktivität wenigstens eines Enzyms aufweist, das eine der Reaktionen der β-Oxidation von Fettsäuren katalysiert. Weiterhin betrifft die Erfindung die Verwendung einer stoffwechselaktiven Zelle, die eine ihrem Wildtyp gegenüber verringerte Aktivität eines Enzyms aufweist, das eine der Reaktionen der β-Oxidation von Fettsäuren katalysiert ausgewählt aus der Gruppe umfassend FadL und FadE sowie Varianten davon, bevorzugter FadE, zur verbesserten Abtrennung einer hydrophoben organischen Lösung von einem wässrigen Kulturmedium umfassend die stoffwechselaktive Zelle.

Ein grundlegendes Problem bei Verfahren zur Herstellung von Feinchemikalien ausgehend von nachwachsenden Rohstoffen anstatt von fossilen Brennstoffen, besteht darin, das einmal erhaltene Produkt, das typischerweise zunächst in einer großvolumigen wässrigen Phase vorliegt, in eine hydrophobe organische Phase zu überführen. Diese Überführung ist einerseits erforderlich, um ein fertiges Zwischen- oder Endprodukt zu konzentrieren und um ggf. die synthetische Verarbeitung in folgenden Reaktionsschritten in organischer Lösung zu ermöglichen, andererseits, um die Ausbeute der Reaktion in der wässrigen Phase durch das Entfernen des erwünschten Produktes zu verbessern oder den Ablauf der Reaktion in einem technisch sinnvollen Rahmen überhaupt erst zu ermöglichen, insbesondere dann, wenn sich die Anwesenheit des Produktes aufgrund einer Toxizität gegenüber dem Produktionsstamm oder einer Inhibition eines relevanten Biokatalysators durch das Produkt nachteilig auf den Reaktionsfortschritt auswirkt. Die direkte thermische Aufkonzentrierung des häufig in niedrigen Konzentrationen vorliegenden Produktes aus der großvolumigen wässrigen Lösung ist in der Regel nicht sinnvoll.

Ein Beispiel für ein solches industriell stark nachgefragtes Produkt, das herkömmlich ausgehend von im Erdöl enthaltenen Kohlenwasserstoffen hergestellt wird, ist 12-Aminolaurinsäure (ALS) bzw. deren Methylester (ALSME). ALS ist ein wichtiges Ausgangsprodukt bei der Herstellung von Polymeren, beispielsweise zur Herstellung von Leitungssystemen auf Nylonbasis. Herkömmlich wird ALS ausgehend von fossilen Rohstoffen in einem Prozess mit niedriger Ausbeute über Laurinlactam hergestellt, das durch Trimerisierung von Butadien, anschließende Hydrierung unter Bildung von Cyclododecan, anschließende Oxidation zu Cyclododecanon, Umsetzung mit Hydroxylamin und anschließender Beckmann-Umlagerung synthetisiert wird. Ein vielversprechender neuer Weg zur biotechnologischen Herstellung von ALS bzw. ALSME ist in der WO 2009/077461 beschrieben. Das diesem Weg zu Grunde liegende biotechnologische Verfahren kann in einem zweiphasigen System unter Verwendung eines flüssigen Ionenaustauschers durchgeführt werden, wie in der EP11154707 beschrieben ist.

Die Aufarbeitung eines Produktes aus einer wässrigen Phase über eine Extraktion in eine hydrophobe organische Phase setzt zunächst voraus, dass dieses Produkt eine ausreichende Neigung aufweist, in einem Zweiphasensystem umfassend eine wässrige, hydrophile Phase und eine organische, hydrophobe Phase, die sich nicht mischen, in die organische Phase einzutreten, was maßgeblich von den physikochemischen Eigenschaften der jeweiligen Verbindung abhängt. Während Verbindungen mit einem hohen Anteil an oder ausschließlich bestehend aus unsubstituierten Kohlenwasserstoffen sich überwiegend in der hydrophoben Phase anreichern, liegen Verbindung mit einem hohen Anteil an polaren Gruppen wie heteroatomhaltigen Funktionalitäten und ganz besonders Verbindungen mit Ladungen überwiegend oder praktisch ausschließlich in der wässrigen Phase vor, was eine Überführung in eine organische Phase erschwert.

Die Verteilung einer Verbindung in dem genannten Zweiphasensystem nach Einstellung des Gleichgewichts wird häufig mit Hilfe von Verteilungskoeffizienten, beispielsweise nach der Nernst'schen Gleichung

$$\alpha = c_{\text{Phase 1}} / c_{\text{Phase 2,}}$$

beschrieben. Ein spezieller Verteilungskoeffizient ist $K_{\text{ow,}}$ auch als P-Wert bezeichnet, der das Verteilungsgleichgewicht einer Verbindung zwischen einer Oktanol- und einer wässrigen Phase charakterisiert:

$$K_{\text{ow}} = P = c_{\text{Oktanol}} / c_{\text{Wasser}}$$

Eine weitere Voraussetzung für die Aufarbeitung des Produktes aus der hydrophoben Phase besteht darin, dass die Verteilung den Gleichgewichtszustand erreicht hat, der durch die vorgenannten Gleichungen beschrieben ist, oder ihm zumindest ausreichend nahekommt. Die Einstellung des Gleichgewichts wird u.a. durch Größe der Kontaktfläche zwischen beiden Phasen bestimmt, ein Faktor, der im Falle biotechnologischer Verfahren in der Regel nicht limitierend ist, da die Kontaktfläche durch Maßnahmen wie die Belüftung und das Durchrühren des wässrigen Kulturmediums und der hydrophoben organischen Lösung, die durch Erhaltung hoher Dichten stoffwechselaktiver Zellen ohnehin notwendig sind, bereits erhöht ist.

Bevor ein solches Reaktionsgemisch, in dem die hydrophobe organische Lösung überwiegend in Micellen oder anderen Subkompartimenten vorliegt, effizient aufgearbeitet werden kann, ist jedoch eine Trennung der beiden Phasen erforderlich. Erfolgt die Ausbildung zweier Phasen beim Mischen von reinem Wasser mit einem reinen organischen hydrophoben Lösungsmittel häufig spontan und ohne weiteres Zutun, so stellt sich die Abtrennung einer organischen hydrophoben Lösung aus einem komplexen wässrigen Kulturmedium aufgrund der vielfältigen möglichen Wechselwirkungen weniger einfach dar und kann ohne technische Unterstützung, beispielsweise durch Zentrifugation, mehrere Stunden oder gar Tag andauern.

Für die großtechnische Produktion von industriell nachgefragten chemischen Verbindungen über biotechnologische Prozesse ist der Vorgang der Phasenabtrennung bei der Entwicklung und Anwendung von Verfahren zur ressourcensparenden und schnellen Produktion und Aufarbeitung von Verbindungen wie ALS vor diesem Hintergrund ein Faktor von erheblicher Bedeutung. Muss das Produkt aus einer großvolumigen wässrigen Phase in einem Großreaktor regelmäßig in einer hydrophoben organischen Lösung gelöst entnommen und anschließend aufbereitet werden, so ist neben den für die eigentliche Herstellung relevanten Parametern wie Art und Menge von Substraten, Temperatur und Sauerstoffgehalt des Mediums auch die Abtrennung der hydrophoben Lösung zu optimieren, um Ressourcen einzusparen und den Gesamtprozess möglichst umweltfreundlich zu gestalten. Nicht zuletzt ist zu erwähnen, dass zahlreiche großtechnisch verwendete hydrophobe organische Lösungsmittel auf biotechnologisch verwendete Organismen zumindest bei längerem Kontakt toxisch wirken und auch vor diesem Hintergrund zur Schonung der biotechnologisch verwendeten Stämme und um zu verhindern, dass unerwünschte bei der Lyse von Zellen freigesetzte Nebenprodukte das Zielprodukt verunreinigen oder gar zersetzen, wünschenswert ist, den der Kontakt zwischen Organismus im wässrigen Kulturmedium und Lösungsmittel zu verkürzen.

Der vorliegenden Erfindung liegt daher die Aufgabe zugrunde, ein Verfahren zur verbesserten Herstellung und Produktion von biotechnologisch erzeugten Produkten in einem biphasischen System umfassend ein wässriges Kulturmedium mit einer stoffwechselaktiven Zelle und eine hydrophobe organische Lösung bereitzustellen. Insbesondere stellt sich die Aufgabe, die Abtrennung der hydrophoben organischen Lösung und die Extraktion eines darin löslichen biotechnologisch erzeugten Produktes mit Hinblick auf die Schnelligkeit des Prozesses bzw. das Ausmaß der Abtrennung der hydrophoben organischen Lösung von dem wässrigen Kulturmedium in einem gegebenen Zeitfenster zu verbessern.

Eine weitere der vorliegenden Erfindung zugrunde liegende Aufgabe besteht darin, eine biotechnologisch verwendbare Zelle mit hoher Resistenz gegen die von hydrophoben Lösungen ausgehende Toxizität gegenüber einer solchen Zelle bereitzustellen.

Weiterhin liegt der vorliegenden Erfindung die Aufgabe zu Grunde, ein biotechnologisches Verfahren zur Herstellung hydrophober Verbindungen, bei dem der Sauerstoffverbrauch reduziert ist, und eine dazu geeignete Zelle bereitzustellen.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch ein Verfahren umfassend die Schritte

a) Bereitstellen eines wässrigen Kulturmediums umfassend eine stoffwechselaktive Zelle,

b) Kontaktieren des wässrigen Kulturmediums mit einer hydrophoben organischen Lösung,

c) Abtrennen der hydrophoben organischen Lösung von dem wässrigen Kulturmedium,

wobei die Zelle eine ihrem Wildtyp gegenüber verringerte Aktivität wenigstens eines Enzyms aufweist, das eine der Reaktionen der β-Oxidation von Fettsäuren katalysiert eine rekombinante Alkanhydroxylase des alkB-Typs aufweist, wobei es sich bei der Zelle um eine prokaryotische Zelle handelt wobei die Verringerung der Aktivität 90 oder mehr Prozent der Wildtyp-Aktivität ist, wobei es sich bei dem Enzym, das eine der Reaktionen der β-Oxidation von Fettsäuren katalysiert, um ein Enzym aus der Gruppe, die FadE und FadL sowie Varianten davon umfasst, bevorzugt um FadE oder eine Variante davon wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen,wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst.

In einer zweiten Ausführungsform des ersten Aspektes handelt es sich um ein Verfahren, wobei die organische Lösung eine Fettsäure mit mehr als 12 Kohlenstoffatomen oder einen Ester davon umfasst, bevorzugt Ölsäure, Erukasäure oder Laurinsäuremethylester.

Die der Erfindung zu Grunde liegende Aufgabe wird in einem zweiten Aspekt gelöst durch die Verwendung einer stoffwechselaktiven prokaryotischen Zelle, die eine ihrem Wildtyp gegenüber verringerte Aktivität eines Enzyms aufweist, das eine der Reaktionen der $\beta$-Oxidation von Fettsäuren katalysiert, bevorzugt eines Enzyms ausgewählt aus der Gruppe umfassend FFadE und FadL sowie Varianten davon, bevorzugter FadE, aufweisend eine rekombinante Alkanhydroxylase des alkB-Typs,

wobei die Verringerung der Aktivität 90 oder mehr Prozent der Wildtyp-Aktivität ist,

wobei es sich bei dem Enzym, das eine der Reaktionen der $\beta$-Oxidation von Fettsäuren katalysiert, um ein Enzym aus der Gruppe FadE und FadL oder deren Varianten davon, wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen,

zur Verbesserung der Abtrennung einer hydrophoben organischen Lösung von einem wässrigen Kulturmedium umfassend die stoffwechselaktive Zelle,

wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst zur Verbesserung der

Abtrennung einer hydrophoben organischen Lösung von einem wässrigen Kulturmedium umfassend die stoffwechselaktive Zelle wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst.

Die der Erfindung zu Grunde liegende Aufgabe wird in einem dritten Aspekt gelöst durch die Verwendung eines Knock outs eines Enzyms, das eine der Reaktionen der $\beta$-Oxidation von Fettsäuren katalysiert, bevorzugt einem Enzym ausgewählt aus der Gruppe umfassend FadE und FadL sowie Varianten davon, noch bevorzugter FadE oder einer Variante davon, wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen, als Teil der genetischen Ausstattung einer stoffwechselaktiven prokaryotischen Zelle aufweisend eine rekombinante Alkanhydroxylase des alkB-Typs, zur Verbesserung der Abtrennung einer hydrophoben organischen Lösung von einem wässrigen Kulturmedium umfassend die stoffwechselaktive Zelle, wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst.

Die der Erfindung zu Grunde liegende Aufgabe wird in einem vierten Aspekt gelöst durch eine prokaryotischche Zelle, die eine ihrem Wildtyp gegenüber verringerte Aktivität eines Enzyms aufweist, das eine der Reaktionen der $\beta$-Oxidation von Fettsäuren katalysiert, weiter umfassend eine rekombinante Alkanhydroxylase des alkB-Typs,

wobei die Verringerung der Aktivität 90 oder mehr Prozent der Wildtyp-Aktivität ist, wobei es sich bei dem Enzym, das eine der Reaktionen der $\beta$-Oxidation von Fettsäuren katalysiert, um ein Enzym aus der Gruppe handelt, die FadE und FadL sowie Varianten davon umfasst, bevorzugter um FadE oder eine Variante davon wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen.

Die der Erfindung zu Grunde liegende Aufgabe wird in einem fünften Aspekt gelöst durch eine Reaktionsmischung umfassend eine wässrige Lösung mit einer stoffwechselaktiven Zelle gemäß oben, und eine hydrophobe organische Lösung, wobei die hydrophobe organische Lösung wenigstens ein Lösungsmittel aus der Gruppe umfasst, die bei Raumtemperatur flüssige, substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst.

In einer ersten Ausführungsform des fünften Aspektes wird die Aufgabe gelöst durch eine Reaktionsmischung, wobei die hydrophobe organische Lösung eine Fettsäure mit mehr als 12 Kohlenstoffatomen oder einen Ester davon umfasst, bevorzugt Ölsäure, Erukasäure oder Laurinsäuremethylester ist.

In einer weiteren Ausführungsform des ersten, zweiten, dritten, vierten oder fünften Aspektes wird die Aufgabe gelöst durch ein Verfahren, eine Verwendung oder eine Reaktionsmischung, wobei es sich bei der stoffwechselaktiven Zelle um eine Zelle handelt, die eine rekombinante Alkanhydroxylase und eine Transaminase aufweist, bevorzugt darüber hinaus wenigstens ein Enzym aus der Gruppe umfassend Alkoholdehydrogenase, Alanindehydrogenase und Lactamhydrolase.

In einer weiteren Ausführungsform des ersten, zweiten, dritten, vierten oder fünften Aspektes wird die Aufgabe gelöst durch ein Verfahren, eine Verwendung oder eine Reaktionsmischung, wobei es sich bei der Zelle um eine prokaryotische Zelle handelt, bevorzugt um *E. coli.*

In einer weiteren Ausführungsform des ersten, zweiten, dritten, vierten oder fünften Aspektes wird die Aufgabe gelöst durch ein Verfahren, eine Verwendung oder eine Reaktionsmischung, wobei es sich bei der Zelle um eine rekombinante Zelle handelt.

In einer weiteren Ausführungsform des ersten, zweiten, dritten, vierten oder fünften Aspektes wird die Aufgabe gelöst durch ein Verfahren, eine Verwendung oder eine Reaktionsmischung, wobei die hydrophobe organische Lösung wenigstens 5, bevorzugt wenigstens 20 Prozent des Gesamtvolumens von hydrophober organischer Lösung und wässrigem Kulturmedium ausmacht.

In einer weiteren Ausführungsform des ersten, zweiten, dritten, vierten oder fünften Aspektes wird die Aufgabe gelöst durch ein Verfahren, eine Verwendung oder eine Reaktionsmischung, wobei der pH-Wert des wässrigen Kulturmediums zum Zeitpunkt des Kontaktierens zwischen 5 bis 9, bevorzugter 6 bis 8 liegt.

Die Erfinder der vorliegenden Erfindung haben festgestellt, dass sich eine hydrophobe organische Lösung überraschend von einem wässrigen Kulturmedium umfassend eine stoffwechselaktive Zelle abtrennen lässt, wenn die stoffwechselaktive Zelle eine ihrem Wildtyp gegenüber verringerte Aktivität wenigstens eines Enzyms aufweist, das eine der Reaktionen der β-Oxidation von Fettsäuren katalysiert.

Weiterhin haben die Erfinder der vorliegenden Erfindung überraschend gefunden, dass eine stoffwechselaktive Zelle, die eine ihrem Wildtyp gegenüber verringerte Aktivität wenigstens eines Enzyms aufweist, das eine der Reaktionen der β-Oxidation von Fettsäuren katalysiert, bei einem biotechnologischen Verfahren bei vergleichbarer Produktionsleistung mit Hinblick auf das Zielprodukt einen geringeren Sauerstoffbedarf und ein höheres Verhältnis an Produktausbeute zu Sauerstoffverbrauch aufweist.

Ohne an irgendeine Theorie gebunden sein zu wollen, vermuten die Erfinder, dass die verringerte Aktivität wenigstens eines Enzyms, das eine der Reaktionen der β-Oxidation katalysiert, dazu führt, dass die Konzentration bisher unidentifizierter Metaboliten, die als Detergentien wirken, im wässrigen Kulturmedium verringert wird, so dass einerseits die Kontaktfläche zwischen hydrophober Lösung und den im wässrigen Kulturmedium befindlichen, lösungsmittelempfindlichen stoffwechselaktiven Zellen beim Kultivieren von Zellen unter Rühren des Mediums verringert wird, was zur Verringerung des Lösungsmittelstresses für die Zellen führt, und andererseits die Ausbildung von getrennten Phasen nach einem Abschalten der Rührvorrichtung gefördert wird.

Die erfindungsgemäße Lehre kann zur Verbesserung sämtlicher biotechnologischer Verfahren herangezogen werden, die die Herstellung von Produkten wie Feinchemikalien unter Verwendung einer stoffwechselaktiven prokaryotische Zelle, deren Kultivierung in einem wässrigen Medium und die Aufarbeitung des Produktes unter Verwendung einer hydrophoben organischen Lösung umfassen. In einer bevorzugten Ausführungsform wird unter dem Begriff "stoffwechselaktive

Zelle", wie hierin verwendet, eine lebende prokaryotische Zelle mit Stoffwechselaktivität verstanden, bevorzugt eine Zelle, die ein für die biotechnologische Herstellung des interessierenden Produktes relevantes Enzym in aktiver Form exprimiert oder noch bevorzugter überexprimiert. Bei der Zelle kann es sich um einen Prokaryonten, einschließlich Archaeen, bevorzugt aus der Gruppe von Gattungen umfassend *Pseudomonas, Corynebacterium* und *Escherichia.* In einer noch bevorzugteren Ausführungsform handelt es sich bei der Zelle um eine bakterielle Zelle, noch bevorzugter um eine Gram-negative bakterielle Zelle, am bevorzugtesten um *E. coli.* Der Begriff "Zelle" wird, in einer besonderen Ausführungsform, in dieser Anmeldung gleichbedeutend und austauschbar mit dem Begriff "Mikroorganismus" verwendet. Weiterhin kann es sich bei der Zelle um eine isolierte Zelle oder eine Mischung verschiedener Zellen handeln.

Dem Fachmann sind zahlreiche wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässriges Kulturmedium", wie hierin verwendet, ein Reaktionsmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, erhält oder fördert und sowohl wässriges Kulturmedium als auch hydrophobe organische Phase in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 0 und 45 °C, bevorzugter zwischen 15 und 40 °C, am bevorzugtesten zwischen 20 und 37 °C.

Dem Fachmann sind zahlreiche Lösungsmittel bekannt, die verwendet werden können, um eine hydrophobe organische Lösung herzustellen. In einer bevorzugten Ausführungsform wird unter dem Begriff "hydrophob", wie hierin verwendet, die Eigenschaft einer Flüssigkeit verstanden, im flüssigen Zustand in Anwesenheit von einer flüssigen wässrigen Phase eine eigene, von der wässrigen Phase klar abgegrenzte flüssige Phase auszubilden. Bei letzterer kann es sich um eine zusammenhängende flüssige Phase oder um eine Emulsion handeln. In einer weiteren bevorzugten Ausführungsform wird mit dem Begriff "hydrophob", wie hierin verwendet, die Eigenschaft einer Verbindung verstanden, sich im Wesentlichen nicht in Wasser zu lösen. Schließlich wird der Begriff in einer weiteren bevorzugten Ausführungsform, wie hierin verwendet, derart verstanden, dass eine derartige bezeichnete Verbindung einen P-Wert (J. Sangster, Octanol-Water Partition Coefficients: Fundamentals and Physical Chemistry, Vol. 2 of Wiley Series in Solution Chemistry, John Wiley & Sons, Chichester, 1997) aufweist, dessen dekadischer Logarithmus größer 0, bevorzugter größer 0,5, noch bevorzugter größer 1 und am bevorzugtesten größer 2 ist. Die organischen Lösungsmittel umfassen Lösungsmittel aus der Gruppe umfassend bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst. Die hydrophobe organische Lösung kann auch eine Mischung umfassend mehr als ein hydrophobes organisches Lösungsmittel sein.

Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen (Fujita *et al.*, 2007). Im weiteren Sinn beginnt sie mit der Aufnahme einer Fettsäure in die Zelle, im Falle von *E. coli* durch den Transporter FadL (Black, 1991), der sie durch die äußere bzw. innere Membran der Gram-negativen Bakterienzelle schleust und das FadD -Genprodukt (Black *et al.*, 1992), das die Fettsäure in Form des CoA-Esters ins Cytosol freisetzt. Dort wird die Fettsäure, sofern die Bedingungen es erfordern, zunächst an der β-Position des CoA-Fettsäureesters durch eine Acyl-CoA-Dehydrogenase, im Falle von *E. coli* FadE (Campbell and Cronan, 2002), oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. Unter dem Begriff "Enzym, das eine der Reaktionen der β-Oxidation von Fettsäuren katalysiert" wird ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. Unter dem Begriff "Enzym, das eine der Reaktionen der β-Oxidation von Fettsäuren katalysiert", wie hierin verwendet, jedes Enzym aus der Gruppe verstanden, die die Genprodukte FadL und FadE aus *E. coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidsequenzen sind im Stand der Technik beschrieben, beispielsweise FadA unter Zugangsnummer AP009048.1, FadB unter Zugangsnummer BAE77457.1, FadD unter Zugangsnummer BAA15609.1, FadE unter Zugangsnummer BAA77891.2 und FadL unter Zugangsnummer BAA16205.1.

Die Lehre der vorliegenden Erfindung kann nicht nur unter Verwendung der bzw. auf die exakten Aminosäure- oder Nukleinsäuresequenzen der hierin beschriebenen biologischen Makromoleküle ausgeführt bzw. angewendet werden, beispielsweise durch Knock out eines Gens, das für ein eine der Reaktionen der β-Oxidation katalysierendes Enzym kodiert, sondern auch unter Verwendung von bzw. auf Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den $K_M$- und/oder $k_{cat}$- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Fettsäureimporter, als Enoyl-CoA-Hydratase bzw. FadE oder als Acetyl-CoA-Acyltransferase bzw. FadB. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* 1995 beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Mit der Entwicklung moderner genetischer, mikrobiologischer und molekularbiologischer Methoden stehen dem Fachmann zahlreiche Werkzeuge zur Verfügung, mit denen er die Aktivität von in lebenden Zellen vorhandenen Enzymen routinemäßig messen und beeinflussen kann. Zur Bestimmung der Aktivität eines Enzyms, die in Form einer Suspension, eines Pellets vorliegt oder in prozessierter Form einer Zellkultur entnommen sein kann, können enzymatische Standardtests verwendet und ausgewertet werden, wie es in Lehrbüchern, beispielsweise Cornish-Bowden, 1995, beschrieben ist. Der Stand der Technik offenbart zahlreiche Tests, die speziell für die Messung der Aktivität von Enzymen geeignet sind, die eine der Reaktionen der β-Oxidation von Fettsäuren katalysieren, beispielsweise in Kameda & Nunn (1981), Marrakchi *et al.* (2003), Lobo *et al.* (2001) und Xu *et al.* (2011). Auch routinemäßig anwendbare Verfahren zur Verringerung der Aktivität eines Enzyms in einer Zelle, beispielsweise durch ungerichtete Mutagenese von Zellen durch Exposition gegenüber radioaktiver Strahlung gefolgt von Anreicherung oder Screening der Mutanten, durch ortsgerichtete Einführung von Punktmutationen oder durch den Knock-out eines chromosomal in eine Zelle integrierten für ein aktives Enzym kodierendes Gen sind im Stand der Technik beschrieben, beispielsweise in Maniatis *et al* (1989) oder in Fuchs & Schlegl (2007). Im besonderen Fall der Fad-Genprodukt bietet sich zu Verringerung der Aktivität auch die Überexpression eines transkriptionellen Repressors, beispielsweise von FadR (Fujita *et al*., 2007). an. Auch eine Aktivitätsverringerung auf der Basis von RNA-Interferenz (Tuschl, 2001) oder unter Verwendung von spezifischen Inhibitoren ist möglich. In einer bevorzugten Ausführungsform bedeutet die Formulierung "wobei die Zelle eine ihrem Wildtyp gegenüber verringerte Aktivität" eines Enzyms aufweist, wie hierin verwendet, dass die Aktivität des Enzyms in der veränderten Zelle gegenüber der Aktivität des gleichen Enzyms in einer Wildtyp-Zelle verringert ist. Die relative Verringerung beträgt in der Reihenfolge zunehmender Bevorzugung 90, 95, 99 oder mehr Prozent der Aktivität. In einer besonders bevorzugten Ausführungsform ist keine Aktivität des Enzyms gegenüber dem Hintergrund mehr nachweisbar.

Beim zweiten Schritt des erfindungsgemäßen Verfahrens kommt es zum Kontaktieren des wässrigen Kulturmediums mit einer hydrophoben organischen Lösung. In einer bevorzugten Ausführungsform der vorliegenden Erfindung bedeutet der Begriff "Kontaktieren", wie hierin verwendet, dass wässriges Kulturmedium und organische Lösung direkt in Kontakt gebracht werden, ohne dass eine mechanische, für ein wässriges Kulturmedium und/oder eine hydrophobe organische Lösung unüberwindbare Barriere, beispielsweise eine anorganische Membran, zwischengeschaltet ist. Beispielsweise kann das wässrige Kulturmedium in einem Fermenter vorgelegt werden, und die organische Lösung wird in den gleichen Fermenter zu dem Kulturmedium gegeben, so dass sich beide Flüssigkeiten vermischen können. In einer bevorzugten Ausführungsform findet das Kontaktieren zumindest teilweise unter Rühren, dem Einströmen von Gas oder ähnlichen Maßnamen statt, die geeignet sind, um die Kontaktfläche beider Phasen zu vergrößern.

Nach dem zweiten Schritt wird die hydrophobe organische Lösung von dem wässrigen Kulturmedium abgetrennt. Dies ist aufgrund der inhärenten Fähigkeit dieses Systems zur Ausbildung zweier Phasen ein für den Fachmann leicht durchzuführender Vorgang, der einfach durch Stehenlassen des Gefäßes und anschließendes Abdekantieren einer Phase vonstatten gehen kann. Alternativ kann ein Scheidetrichter Verwendung finden. Im Falle ausreichend verschiedener Siedepunkte besteht die Möglichkeit, die bei niedrigeren Temperaturen siedende Phase, bei der es sich in der Regel um die organische Phase handeln wird, durch das Anlegen von Unterdruck abzuziehen. Geringe Mengen von in der organischen Phase verbliebenem Wasser können unter Verwendung von anorganischen Trockenmitteln wie Calciumhydrid, wasserfreiem Calciumchlorid, Kieselgel, wasserfreiem Natriumsulfat, Natriumhydroxid oder dergleichen entfernt werden.

Das erfindungsgemäße Verfahren kann unter Verwendung üblicher hydrophober organischer Lösungsmittel durchgeführt werden, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfassen. Auch hydrophobe organische Lösungsmittel, die *per se* nicht flüssig sind, eignen sich, sofern sie Teil eines Gemisches von Lösungsmitteln sind, das in seiner Gesamtheit flüssig ist. Zu berücksichtigen ist dabei gegebenenfalls, dass zahlreiche Lösungsmittel auf stoffwechselaktive Zellen mehr oder weniger toxisch wirken. Soll die Zelle ihre Stoffwechselaktivität also wenigstens zeitweilig behalten, so sind entsprechende mäßig oder gar nicht toxisch wirkende Konzentrationen des hydrophoben organischen Lösungsmittels zu verwenden. In einer besonders bevorzugten Ausführungsform handelt es sich bei dem Lösungsmittel um eine gesättigte oder ungesättigte Fettsäure mit wenigstens acht, bevorzugt wenigstens zwölf Kohlenstoffatomen, beispielsweise um Laurinsäure, Ölsäure oder Erukasäure oder die Methylester davon. In einer weiteren bevorzugten Ausführungsform handelt es sich bei dem Lösungsmittel mit eine Fettsäure der Formel $CH_3 - (CH_2)_x - COOH$, wobei x 8, 9, 10, 11, 12, 13, 14, 15, 16, 17, 18, 19, 20, 21, 22 oder mehr sein kann. In einer weiteren bevorzugten Ausführungsform handelt es sich um eine ungesättigte Fettsäure mit einer Doppelbindung, besonders bevorzugt um eine an der Position 9, besonders bevorzugt Ölsäure. In einer weiteren besonders bevorzugten Ausführungsform handelt es sich bei dem Lösungsmittel um Hexansäure.

Das Volumen der hydrophoben organischen Lösung sollte so bemessen sein, dass die organische Phase leicht abgetrennt werden kann und beträgt in einer bevorzugten Ausführungsform 2 bis 98, bevorzugter 5 bis 95, noch bevorzugter 10 bis 40, am bevorzugtesten 20 bis 30 Prozent des Gesamtvolumens von wässrigem Kulturmedium und hydrophober organischer Lösung.

Als stoffwechselaktive Zelle wird in der Regel eine Zelle gewählt werden, die ein Produkt von besonderem Interesse herzustellen vermag. Besonders vorteilhaft ist dafür die Verwendung einer rekombinanten Zelle. In einer bevorzugten Ausführungsform wird unter dem Begriff "rekombinant", wie hierin verstanden, dass das als rekombinant bezeichnete, in die "rekombinate Zelle" eingebrachte, Nukleinsäuremolekül ein nicht der Natur entnommenes, sondern ein unter Verwendung von molekularbiologischen oder chemisch-synthetischen Verfahren hergestelltes Nukleinsäuremolekül ist bzw. dass die als rekombinant bezeichnete Zelle ein rekombinantes Nukleinsäuremolekül oder ein davon kodiertes Polypeptid umfasst. Molekularbiologische Routineverfahren zum Herstellen rekombinanter Nukleinsäuremoleküle und Zellen sind im Stand der Technik beschrieben, beispielsweise in Sambrook *et al.* (1989) oder Schlegl & Fuchs (2007).

Besonders vorteilhaft ist es weiterhin, wenn die Zelle ein ein Produkt oder eine Vor- oder Zwischenstufe davon herstellendes Enzym aufweist oder, besonders bevorzugt, überexprimiert. Dies kann erreicht werden, indem ein Vektor, der ein für das Enzym kodierende Nukleinsäuremolekül umfasst, durch Transformation o.ä. in die Zelle eingeschleust wird oder das für das Enzym kodierendes Nukleinsäuremolekül in die genetische Ausstattung der Zelle, beispielsweise ein Chromosom, eingebaut wird. Für zahlreiche biotechnologisch bedeutsame Arten von Zellen, z.B. *E. coli,* sind geeignete Verfahren und Vektoren bekannt, die für die Expression oder Überexpression eines Nukleinsäuremoleküls verwendet werden können, beispielsweise die Vektoren vom Typ pET oder pGEX und für deren Expression geeignete Zellen (Moffatt & Studier (1986), Rosenberg *et al.* (1987) und Studier *et al*(1990).

Das erfindungsgemäße Verfahren bietet sich besonders dann an, wenn eine stoffwechselaktive Zelle verwendet wird, um ein hydrophobes organisches Lösungsmittel zu verstoffwechseln oder eine chemische Reaktion unter Verwendung des hydrophoben organischen Lösungsmittels als Substrat zu katalysieren. Zu diesen Enzymen zählen die Alkanhydroxylasen.

In einer besonders bevorzugten Ausführungsform handelt es sich bei einer "Alkanhydroxylase", wie hierin verwendet, um ein Enzym, das die Oxidation eines Alkans zum Alkohol, Aldehyd/Keton und/oder zur Carbonsäure, bevorzugt überwiegend zum Alkohol, katalysiert. Alkanhydroxylasen sind im Stand der Technik zahlreich beschrieben, beispielsweise in Grant *et al.* (2011) oder Koch *et al.* (2009). In einer besonders bevorzugten Ausführungsform handelt es sich bei der Alkanhydroxylase um eine "Alkanhydroxylase des alkB-Typs". AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig. AlkT wird als FAD-abhängige Rubredoxin-Reduktase charakterisiert, die Elektronen von NADH auf AlkG überträgt. AlkG ist ein Rubredoxin, ein eisenhaltiges Redoxprotein, das als direkter Elektronendonor für AlkB fungiert. In einer bevorzugten Ausführungsform wird unter dem Begriff "Alkanhydroxylase des alkB-Typs", wie hierin verwendet, eine membranständige Alkanhydroxylase verstanden. In einer weiteren bevorzugten Ausführungsform wird unter dem selben Begriff "Alkanhydroxylase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1, dieser und sämtliche weitere in diesem Dokument verwendete Datenbankcodes stammen aus der Genbank Protein-Datenbank des NCBI in dem am 9. November 2011 verfügbaren Release) verstanden. Der Begriff "Sequenz", wie hierin verwendet, kann sich auf die Aminosäuresequenz eines Polypeptides und/oder die dafür codierende Nukleinsäuresequenz beziehen.

Das Verfahren kann verwendet werden, um Fettsäuren oder deren Ester zunächst zu oxidieren und anschließend zu aminieren. Dazu eignet sich beispielsweise ein Enzymsystem, wie es in der internationalen Patentanmeldung WO 2009/077461 beschrieben ist. Bei der stoffwechselaktiven Zelle handelt es sich in diesem Fall um eine Zelle, die eine rekombinante Alkanhydroxylase und eine Transaminase aufweist, bevorzugt darüber hinaus wenigstens ein Enzym aus der Gruppe umfassend Alkoholdehydrogenase, Alanindehydrogenase und Lactamhydrolase. In einer bevorzugten Ausführungsform wird unter dem Begriff "Alkoholdehydrogenase", wie hierin verwendet, wird ein Enzym verstanden, das einen Aldehyd bzw. Keton zu dem entsprechenden primären bzw. sekundären Alkohol oxidiert. Beispiele umfassend die Alkoholdehydrogenasen von *Ralstonia eutropha* (ACB78191.1), *Lactobacillus brevis* (YP_795183.1), *Lactobacillus kefiri* (ACF95832.1), aus Pferdeleber, von *Paracoccus pantotrophus* (ACB78182.1) und *Sphingobium yanoikuyae* (EU427523.1) sowie die jeweiligen Varianten davon. In einer bevorzugten Ausführungsform wird unter dem Begriff "Transaminase", wie hierin verwendet, ein Enzym verstanden, das die Übertragung von $\alpha$-Aminogruppen von einem Donor-, bevorzugt einer Aminosäure, auf ein Akzeptormolekül, bevorzugt eine $\alpha$-Ketocarbonsäure, katalysiert. Beispielsweise kann die Transaminase von *Chromobacterium violaceum* ATCC 12472 (Datenbankcode NP_901695) verwendet werden. In einer bevorzugten Ausführungsform wird unter dem Begriff "Alanindehydrogenase", wie hierein verwendet, ein Enzym verstanden, das die Umwandlung von L-Alanin unter Verbrauch von Wasser und $NAD^+$ zu Pyruvat, Ammoniak und NADH katalysiert. Beispielsweise können die Alanindehydrogenasen aus *Bacillus subtilis* (Datenbankcode L20916), *Rhizobium leguminosarum* (Datenbankcode CP001622), *Vibrio proteolytikus* (Datenbankcode AF070716), *Mycobacterium tuberculosis* (Datenbankcode X63069), *Enterobacter aerogenes* (Datenbankcode AB013821) verwendet werden.

Die vorliegende Erfindung betrifft nicht nur das erfindungsgemäße Verfahren, sondern auch die Verwendung eines Knock outs eines Enzyms, das eine der Reaktionen der $\beta$-Oxidation von Fettsäuren katalysiert ausgewählt aus der Gruppe umfassend FadE und FadL, bevorzugter FadE, als Teil der genetischen Ausstattung einer stoffwechselaktiven Zelle zur Verbesserung der Abtrennung einer hydrophoben organischen Lösung von einem wässrigen Kulturmedium umfassend die stoffwechselaktive Zelle. Besteht also im Rahmen eines beliebigen Verfahrens die Notwendigkeit, eine hydrophobe organische Lösung von einem wässrigen Kulturmedium umfassend eine stoffwechselaktive Zelle zu trennen, so ist erfindungsgemäß statt einer stoffwechselaktiven Zelle ohne entsprechende Modifikation des Fettsäurestoffwechsels, also einer Zelle mit gegenüber ihrem Wildtyp unveränderter oder gar erhöhter Aktivität der Enzyme, die eine Reaktion der $\beta$-Oxidation von Fettsäuren katalysieren der Gruppe umfassend FadE und FadL, bevorzugter FadE, eine Zelle zu verwenden, bei der wenigstens ein Enzym, das eine der Reaktionen der $\beta$-Oxidation von Fettsäuren katalysiert, ausgeknockt ist ausgewählt aus der Gruppe umfassend FadE und FadL, bevorzugter FadE.

Dieser Knockout eines Fad-Gens ist unabhängig davon zu verwenden, ob die stoffwechselaktive Zelle zur Herstellung einer Fettsäure oder eines Derivates davon oder von einem anderen Molekül verwendet werden soll, das über den Stoffwechselweg der β-Oxidation abgebaut werden könnte.

In einer bevorzugten Ausführungsform ist unter dem Begriff "Knock-out", wie hierin verwendet, zu verstehen, dass die Transkription und/oder Translation des Gens bzw. seines Genproduktes im Vergleich zur Wildtypzelle verringert ist, beispielsweise durch Deletion eines Teils des oder des ganzen Gens, durch Insertion eines Stoppkodons an geeigneter Stelle, durch Entfernung eines essentiellen Teils des Promoters oder durch Entfernung der Ribosomenbindungsstelle.

In einer bevorzugtesten Ausführungsform umfasst das erfindungsgemäße Verfahren die Schritte a) Bereitstellen eines wässrigen Kulturmediums umfassend eine stoffwechselaktive Zelle, b) Kontaktieren des wässrigen Kulturmediums mit einer hydrophoben organischen Lösung und c) Abtrennen der hydrophoben organischen Lösung von dem wässrigen Kulturmedium, wobei die Zelle eine ihrem Wildtyp gegenüber verringerte Aktivität von FadE oder einer Variante davon aufweist, bei der stoffwechselaktiven Zelle handelt es sich um einen rekombinanten Stamm von *E. coli,* der eine rekombinante Alkanhydroxylase AlkBGT aus *Pseudomonas putida* oder eine Variante davon, sowie eine rekombinante Transaminase aufweist, und das hydrophobe organische Lösungsmittel umfasst eine Mischung aus Laurinsäure oder Hexansäure bzw. dem Methylester davon und einer ungesättigten Fettsäure, bevorzugt Ölsäure oder Erukasäure, am bevorzugtesten Ölsäure, wobei bevorzugt das Verhältnis von Ölsäure oder Erukasäure zu Laurinsäure oder Hexansäure bzw. dem Methylester davon 20:80 bis 80 zu 20, bevorzugt 20:80 bis 40:60 beträgt.

In einer bevorzugtesten Ausführungsform ist das erfindungsgemäße Verfahren ein Verfahren zu Herstellung einer ω-Aminocarbonsäure umfassend die Schritte a) Bereitstellen eines wässrigen Kulturmediums umfassend eine stoffwechselaktive Zelle, b) Kontaktieren des wässrigen Kulturmediums mit einer hydrophoben organischen Lösung, c) Abtrennen der hydrophoben organischen Lösung von dem wässrigen Kulturmedium, wobei die Zelle eine ihrem Wildtyp gegenüber verringerte Aktivität wenigstens eines Enzyms aufweist, das eine der Reaktionen der β-Oxidation von Fettsäuren katalysiert, wobei es sich bei der Zelle bevorzugt um *E. coli* handelt und das Enzym, das eine der Reaktionen der β-Oxidation von Fettsäuren katalysiert, FadE ist, und die Zelle darüber hinaus derart gentechnisch verändert ist, dass sie eine ihrem Wildtyp gegenüber erhöhte Menge an ω-Aminocarbonsäure herstellt, bevorzugt dadurch, dass sie mit einem System rekombinanter Enzyme umfassend eine Alkanhydroxylase AlkBGT aus *Pseudomonas putida,* optional eine Alkoholdehydrogenase und eine ω-Transaminase ausgestattet ist. In einer weiteren bevorzugtesten Ausführungsform betrifft die Erfindung eine Zelle, die eine ihrem Wildtyp gegenüber verringerte Aktivität wenigstens eines Enzyms aufweist, das eine der Reaktionen der β-Oxidation von Fettsäuren katalysiert, wobei es sich bei der Zelle bevorzugt um *E. coli* handelt und das Enzym, das eine der Reaktionen der β-Oxidation von Fettsäuren katalysiert, FadE ist, und die Zelle darüber hinaus derart gentechnisch verändert ist, dass sie eine ihrem Wildtyp gegenüber erhöhte Menge an ω-Aminocarbonsäure herstellt, bevorzugt dadurch, dass sie mit einem System rekombinanter Enzyme umfassend eine Alkanhydroxylase AlkBGT aus *Pseudomonas putida,* optional eine Alkoholdehydrogenase und eine ω-Transaminase ausgestattet ist.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

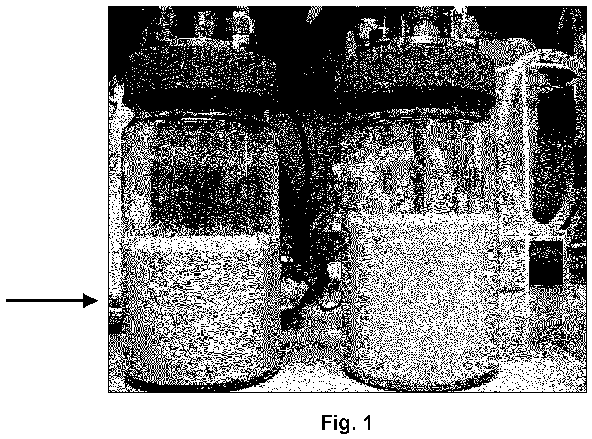

**Fig. 1** zeigt Unterschiede bei der Phasentrennung bei der Herstellung von ALSME unter Verwendung der ΔFadE-Mutante W3110 ΔFadE [alkB-alaD-TA] (im Folgenden auch als "ΔFad" bezeichnet) (links) und des abgesehen vom Fehlen der ΔFadE-Deletion identischen Stammes W3110 [alkB-alaD-TA] (im Folgenden auch als Wildtyp ("WT") bezeichnet) (rechts). Der Pfeil zeigt die bei der Mutante deutliche Trennung der organischen und der wässrigen Phase nach zehn Minuten, wohingegen bei Verwendung des anderen Stammes nach der gleichen Zeit noch keine Phasentrennung erkennbar ist.

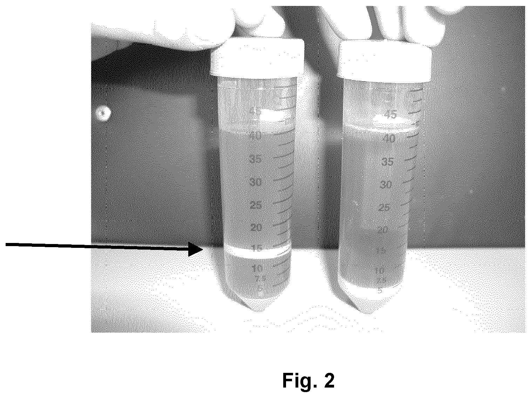

**Fig. 2** zeigt das gleiche Experiment wie **Fig. 1,** abgesehen davon, dass das Reaktionsmedium nach der Fermentation in Falcon-Tubes abgefüllt wurde.

**Fig. 3** zeigt den Sauerstoffeintrag in Form der OTR (oxygen transfer rate) und den Kohlendioxidausstoß in Form der CTR (carbon dioxide transfer rate) beider Stämme bei dem gleichen Experiment wie in **Fig. 1.**

**Fig. 4** zeigt die Konzentrationen von ALSME über die Zeit im Medium bei dem gleichen Experiment wie in **Fig. 1.**

**Fig. 5** zeigt die Konzentration verschiedener Produkte bei der Umsetzung von Laurinsäuremethylester (LSME) wie in Beispiel 2 beschrieben, nämlich Aminolaurinsäure (ALS), Aminolaurinsäuremethylester (ALSME), ω-Carboxylaurinsäure (DDS) w-Carboxylaurinsäuremethylester (DDSME), ω-Hydroxylaurinsäure (HLS), ω-Hydroxylaurinsäuremethylester (HLSME) und ω-Oxolaurinsäure (OLS). Als Stämme wurden der ΔFadE- (**Fig. 5a**), der Wildtyp (W3110) (**Fig. 5b**), der ΔFadL- (**Fig. 5c**) und ein Stamm mit ΔFadE und ΔFadL (**Fig. 5d**) untersucht.

**Fig. 6** zeigt die OTR- (**Fig. 6a**) und CTR- (**Fig. 6b**) Kurven zum in Beispiel 2 beschriebenen Versuch.

**Fig. 7** zeigt Unterschiede bei der Phasentrennung bei der Herstellung von ALSME unter Verwendung des ΔFadE-, Wildtyp (W3110)-, ΔFadL- und ΔFadE/ΔFadL-Stammes in Beispiel 2 beobachtet wurden. Die Pfeile zeigen die bei den Mutanten deutliche Trennung der organischen und der wässrigen Phase nach zehn Minuten, wohingegen bei Verwendung des Wildtyp-Stammes nach der gleichen Zeit noch keine Phasentrennung erkennbar ist.

**Beispiel 1: Beschleunigung der Trennung einer wässrigen von einer hydrophoben Phase unter Verwendung einer ΔFadE-Mutante bei der biotechnologischen Herstellung von Aminlaurinsäuremethylester**

Die Biotransformation von Laurinsäuremethylester zu Aminolaurinsäuremethylester wurde im 8-fach Parallelfermentationssystem von DASGIP mit den Stämmen W3110 ΔFadE [alkB-alaD-TA] und W3110 [alkB-alaD-TA]. Bei W3110 [alkB-alaD-TA] handelt es sich um einen Stamm von *E. coli* W3110, der ein pBR322-basiertes Plasmid mit Oxidations- und Transaminiserungssystem umfassend Oxidoreduktase AlkB, Alanindehydrogenase und Transaminase umfasst, wie es in der WO2009077461 beschrieben ist. W3110 ΔFadE [alkB-alaD-TA] ist mit letzterem Stamm identisch, abgesehen davon, dass das für FadE codierende Gen deletiert ist und der Stamm somit keine Acyl-CoA-Dehydrogenase-Aktivität aufweist.

Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Trinkwasser befüllt und 20 min bei 121°C autoklaviert, um Sterilität zu gewährleisten. Anschließend wurden die pO2-Sonden über Nacht (mindestens 6 h lang) am DASGIP-System polarisiert. Am nächsten Morgen wurde das Wasser unter der Clean Bench entnommen und durch 300 mL Hochzelldichtemedium mit 100 mg/L Ampicillin ersetzt. Im Folgenden wurden die pO2-Sonden mit einer Einpunkt-Kalibration (Rührer: 400 rpm / Begasung: 10 sL/h Luft) kalibriert und die Feed-, Korrekturmittel-und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Die ALS und ALSME produzierenden *E. coli*-Stämme wurden zuerst aus den jeweiligen Cryokulturen in LB-Medium (25 mL in einem 100 mL Schikanekolben) mit 100 mg/L Ampicillin über Nacht bei 37°C und 200 rpm ca. 18 h lang angezogen. Anschließend wurden je 2 mL der Kulturen in Hochzelldichtemedium (Glucose 15 g/L (30 mL / L einer separat autoklavierten 500 g/L Stammlösung mit 1% $MgSO_4*7H_2O$ und 2,2% $NH_4Cl$), $(NH_4)_2SO4$ 1,76 g/L, $K_2HPO_4$ 19,08 g/L, $KH_2PO_4$ 12,5 g/L, Hefeextrakt 6,66 g/L, Trinatriumcitrat-Dihydrat 2,24 g/L, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, $MnCl_2*4H_2O$ 1,91 g/L, $ZnSO_4*7H_2O$ 1,87 g/L, Ethylendiamintetraessigsäure-Dihydrat 0,84 g/L, $H_3BO_3$ 0,30 g/L. $Na_2MoO_4*2H_2O$ 0,25 g/L, $CaCl_2*2H_2O$ 4,70 g/L, $FeSO_4*7H_2O$ 17,80 g/L, $CuCl_2*2H_2O$ 0,15 g/L)) (Je Stamm 25mL in einem 100 mL Schikanekolben) mit 100 mg/L Ampicillin überimpft und bei 37°C / 200 rpm weitere 5,5 h lang inkubiert.

Die optische Dichte der Kulturen bei 600 nm wurde beim W3110 ΔFadE [alkB-alaD-TA] mit 6,9 und beim W3110 [alkB-alaD-TA] mit 7,4 bestimmt. Um die Reaktoren mit einer optischen Dichte von 0,1 anzuimpfen wurden je 4,0 mL bzw. 4,4 mL (ΔFadE) in einer 5 mL Spritze (unter sterilen Bedingungen) aufgezogen und die Reaktoren mittels Kanüle über ein mit 70% Ethanol überschichtetes Septum beimpft.

Folgendes Standardprogramm wurde verwendet:

| DO-Regler | | pH-Regler | |
|---|---|---|---|
| Preset | 0% | Preset | 0 ml/h |
| P | 0,1 | P | 5 |
| Ti | 300 s | Ti | 200 s |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| | 0% | 30% | | 0% | 100% | | 15% | 80% |
| Wachstum und Biotransformation | 400 rpm | 1500 rpm | Wachstum und Biotransformation | 21 % | 21% | Wachstum und Biotransformation | 6 sL/h | 72 sL/h |

| Script | |
|---|---|
| Trigger scharf | 31 % DO (1/60h) |
| Induktion IPTG | 2 h nach Feedstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollen, und die nachfolgende Biotransformation, in der nach Zugabe des Substrates Laurinsäuremethylester eine Umsetzung zu Aminolaurinsäureester von in der Expression gebildeten Enzyme stattfinden soll. Die pH-Werte wurden einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt. Die Fermentation wurde als Fed-Batch durchgeführt, wobei der Feedstart, 5 g/Lh Glucosefeed (500 g/L Glucose mit 1% $MgSO_4*7H_2O$ und 2,2% $NH_4Cl$), über einen DO-Peak getriggert wurde. Mit Feedstart wurde auch die Temperatur von vorher 37°C auf 30°C gesenkt. Die Expression der Transaminase wurde 2 h nach Feedstart durch die automatische Zugabe von IPTG (1 mM) induziert. Die Induktion der alk-Gene erfolgte durch die manuelle Zugabe von DCPK (0,025 % v/v) 10 h nach Feedstart. Vor Start der Biotransformation wurde die optische Dichte der Kulturbrühen bestimmt.

Der Start der Biotransformationsphase erfolgte 14 h nach Feedstart. Dazu wurden 150 mL eines Gemisches aus Laurinsäuremethylesther und dem Ionentauscher Ölsäure (techn. 90%) als Batch zu der Fermentationsbrühe zugegeben. Um einen Aminogruppendonor für die Transaminase zur Verfügung zu stellen, wurde eine halbe Stunde vor Biotransformationsstart 5 mL einer 3M Ammoniumsulfatlösung zu der Fermentationsbrühe zugegeben. Zur Probennahme wurden 2 mL Fermentationsbrühe aus dem Kessel entnommen und ein Teil davon 1/20 in einem Aceton-HCl-Gemisch (c(HCl) = 0,1 mol/L) verdünnt und extrahiert. Proben wurden bei 1 h, 2 h, 3 h, 4 h, 5 h, 7.5 h, 10.5 h, 19.5 h und 21 h nach Start der Biotransformation von allen Reaktoren genommen. Die Umsatzraten für Sauerstoff (OTR = oxygen transfer rate) und Kohlenstoff (CTR = carbon transfer rate) wurden während der Fermentation über die Abgasanalytik an den DASGIP-Systemen bestimmt. Die Fermentation wurde 21 h nach Start der Biotransformation beendet. Der Rührer, die Begasung, die Temperaturregelung und pH-Regelung wurden ausgestellt und die Kessel 5-10 Minuten ruhig stehen gelassen.

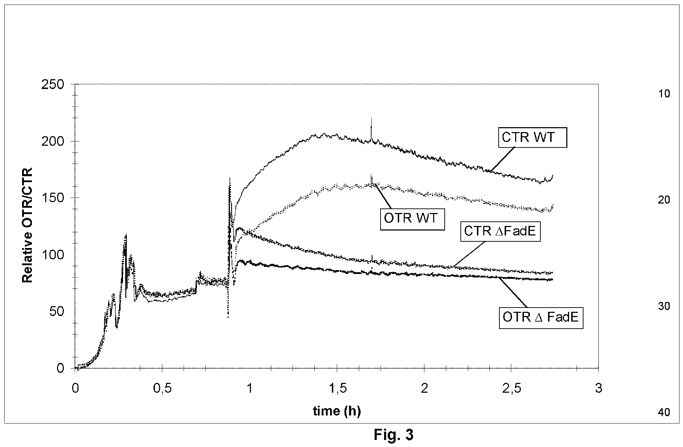

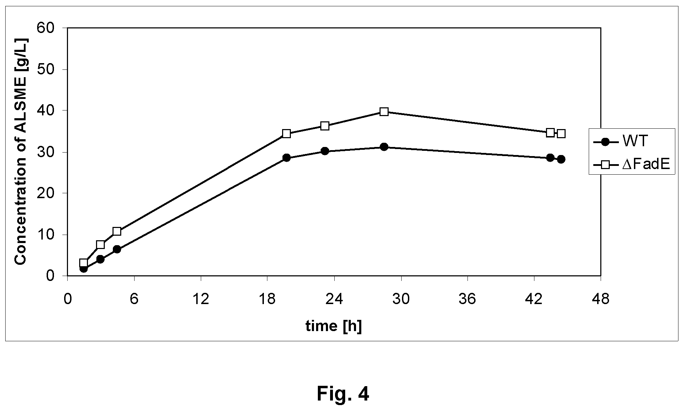

Ergebnisse:
Während der Biotransformationsphase zeigt sich im Falle des W3110 ΔFadE [alkB-alaD-TA] bei vergleichbarer, sogar leicht gesteigerter Bildung des Produktes ALSME ein geringerer Sauerstoffverbrauch als bei W3110 [alkB-alaD-TA] (siehe **Fig. 3** und **4**).

Nach 10 Minuten wurde in dem Kessel mit dem Stamm W3110 ΔFadE [alkB-alaD-TA] eine deutliche Phasentrennung im Verhältnis ca. 40% obere Phase, ca. 60% untere Phase beobachtet. Zwischen den beiden Phasen gab es eine dünne Schicht Interphase. Aus der unteren und oberen Phase wurden Proben in ein 15 mL Falcon-Tube abgefüllt und bei 5500 x g für 10 Minuten abzentrifugiert. Danach zeigte sich, dass sich im Röhrchen mit der unteren Phase zu ca. 95% wässrige Phase und Biomasse befand. Im Röhrchen mit der oberen Phase war zu erkennen, dass die obere Phase aus über 60% Organikanteil bestand. Im Kessel mit dem Stamm W3110 W3110 [alkB-alaD-TA] lag nach 10 Minuten eine homogene Emulsion vor und es zeigte sich auch nach weiteren 20 Minuten Wartezeit keine Phasentrennung.

**Beispiel 2: Beschleunigung der Trennung einer wässrigen von einer hydrophoben Phase unter Verwendung einer ΔFadL-Mutante sowie einer ΔFadE ΔFadL-Mutante bei der biotechnologischen Herstellung von Aminlaurinsäuremethylester**

Analog zu Beispiel 1 wurden weitere Experimente mit den Stämmen

W3110 ΔfadL [alkB-alaD-TA] und

W3110 ΔfadE ΔfadL [alkB-alaD-TA] sowie

W3110 ΔfadE [alkB-alaD-TA] und

W3110 [alkB-alaD-TA]

als Kontrollen durchgeführt.

Wieder wurde das DASGIP 8 fach Parallelfermentationssystem mit exakt dem gleichen Protokoll und den Parametern wie in Beispiel 1 beschrieben verwendet.

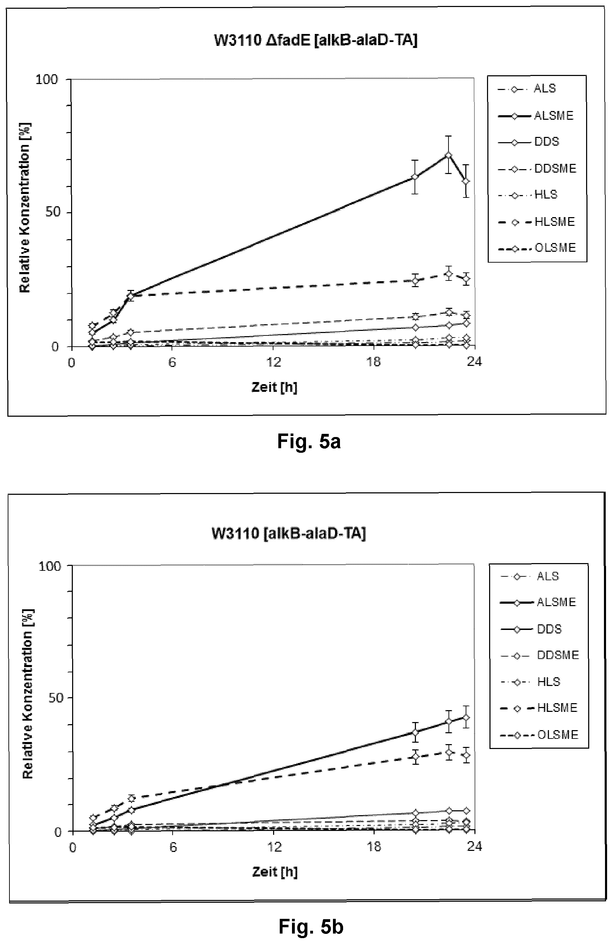

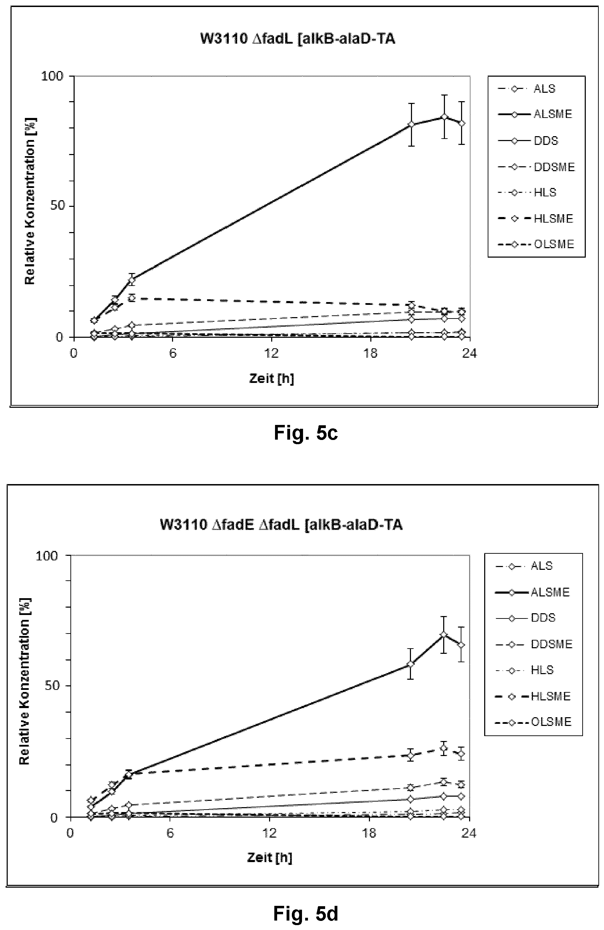

Nach Start der Biotransformation durch Zugabe des Laurinsäuremethylester/Ölsäure-Gemisches wurden nach 1,25, 2,5, 3,5, 20,5, 22,5 und 23,5 Stunden Proben genommen und nach oben genannter Methode aufgearbeitet. Die Ergebnisse sind in den **Figs. 5, 6** und **7** zusammengefasst.

Nach 24 Stunden wurden die Biotransformationen abgebrochen, pH-, Temperatur- und DO- Regelung beendet und die Reaktoren 5 - 10 Minuten ruhig stehen gelassen.

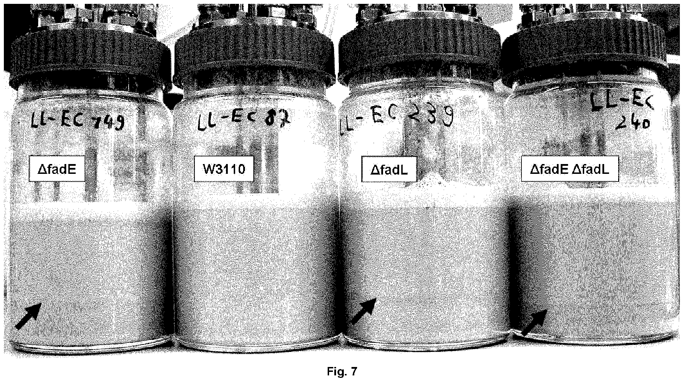

Wie beim oben genannten Experiment konnte auch hier nach kurzer Zeit eine deutliche Trennung der wässrigen von der organischen Phase beobachtet werden, sofern wenigstens eines der Fad-Gene ausgeschaltet war. Im Falle des als Biokatalysator verwendeten Wildtyps, d. h. ohne Deletion eines der Fad-Gene, lässt sich keinerlei Phasentrennung erkennen (siehe **Fig. 7**).

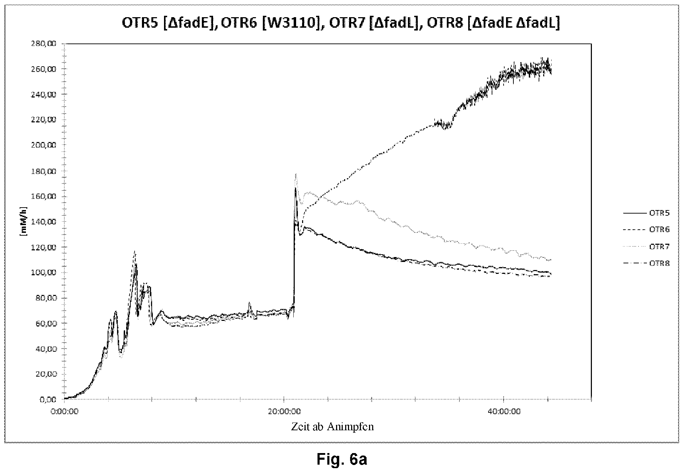

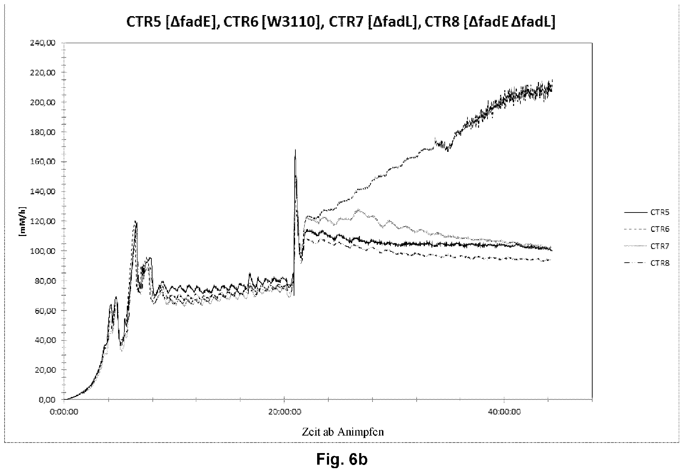

Es zeigte sich ebenfalls, dass die Stämme mit wenigstens einer Fad-Deletion der Sauerstoffverbrauch gegenüber dem Wildtyp reduziert ist (**Fig. 6a**).

**Literaturstellen:**

WO 2009/077461: ω-Aminocarbonsäuren oder ihre Lactame, herstellende, rekombinante Zellen

J Sangster, Octanol-Water Partition Coefficients: Fundamentals and Physical Chemistry, Vol. 2 of Wiley Series in Solution Chemistry, John Wiley & Sons, Chichester, 1997

F M Ausubel (1995), Current Protocols in Molecular Biology. John Wiley & Sons, Inc.

A M Lesk (2008), Introduction to Bioinformatics, 3rd Edition

Y Fujita, H Matsuoka, and K Hirooka (2007) Mol. Microbiology 66(4), 829-839

P N Black (1991) J. Bacteriol. 173, 435-442

P N Black, C C DiRusso, A K Metzger, and T L Heimert (1992) J. Biol. Chem. 267, 25513-25520

J. W Campbell & J E Cronan (200) J. Bacteriol. 184, 3759-3764

A Cornish-Bowden (1995), Fundamentals of Enzym Kinetics, Portland Press Limited, 1995

S Lobo, G Florova, and KA Reynolds (2001) Biochemistry 40 (39), 11955-64

X Yu, T Liu, F Zhu, and C Khosla (2011) PNAS, elektronische Veröffentlichung vor Drucklegung K Kameda & W D Nunn (1981) J. Biol. Chem. 256, 5702-5707

Hi Marrakchi, W E DeWolf, C Quinn, J West, B J Polizzi, C Y So et al. (2003) Biochem. J. 370, 1055-1062

Sambrook/Fritsch/Maniatis (1989): Molecular Cloning: A Laboratory Manual, Cold Spring Harbor Laboratory Press, 2nd edition

Fuchs/Schlegel (2007) Allgemeine Mikrobiologie, 2008, Georg Thieme Verlag

B A Moffatt, and F W Studier (1986) J. Mol. Biol. 189, 113-130

A H Rosenberg, B N Lade, D Chui, S Lin, J J Dunn, and F W Studier (1987) Gene 56, 125-135

F W Studier, A H Rosenberg, J J Dunn, and J W Dubendorff (1990) Meth. Enzymol. 185, 60-89

C Grant, J M Woodley, F Baganz (2011) Enzyme and Microbial Technology, 480-486

D J Koch, M M Chen, J B van Beilen, and F H Arnold (2009) Appl. and Env. Microbiology, 337-344

T Tuschl (2001) ChemBioChem 2: 239-145

**Patentansprüche**

1. Verfahren umfassend die Schritte

   a) Bereitstellen eines wässrigen Kulturmediums umfassend eine stoffwechselaktive Zelle,
   b) Kontaktieren des wässrigen Kulturmediums mit einer hydrophoben organischen Lösung,
   c) Abtrennen der hydrophoben organischen Lösung von dem wässrigen Kulturmedium,

   wobei die Zelle eine ihrem Wildtyp gegenüber verringerte Aktivität wenigstens eines Enzyms aufweist, das eine der Reaktionen der β-Oxidation von Fettsäuren katalysiert,
   eine rekombinante Alkanhydroxylase des alkB-Typs aufweist,
   wobei es sich bei der Zelle um eine prokaryotische Zelle handelt,
   wobei die Verringerung der Aktivität 90 oder mehr Prozent der Wildtyp-Aktivität ist,
   wobei es sich bei dem Enzym, das eine der Reaktionen der β-Oxidation von Fettsäuren katalysiert, um ein Enzym aus der Gruppe FadE und FadL oder deren Varianten davon handelt,
   wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen,
   wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst und
   wobei "hydrophoben organischen Lösung" bedeutet, dass die organische Lösung einen P-Wert aufweist, dessen dekadischer Logarithmus größer 0 ist.

2. Verfahren nach Anspruch 1, wobei die organische Lösung eine Fettsäure mit mehr als 12 Kohlenstoffatomen oder einen Ester davon umfasst, bevorzugt Ölsäure, Erukasäure oder Laurinsäuremethylester.

3. Verwendung einer stoffwechselaktiven, prokaryotischen Zelle, die eine ihrem Wildtyp gegenüber verringerte Aktivität eines Enzyms aufweist, das eine der Reaktionen der β-Oxidation von Fettsäuren katalysiert ausgewählt aus der Gruppe FadE und FadL und Varianten davon
   aufweisend eine rekombinante Alkanhydroxylase des alkB-Typs,
   wobei die Verringerung der Aktivität 90 oder mehr Prozent der Wildtyp-Aktivität ist,
   wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen,
   zur Verbesserung der Abtrennung einer hydrophoben organischen Lösung von einem wässrigen Kulturmedium umfassend die stoffwechselaktive Zelle,
   wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst und
   wobei "hydrophoben organischen Lösung" bedeutet, dass die organische Lösung einen P-Wert aufweist, dessen dekadischer Logarithmus größer 0 ist.

4. Verwendung eines Knock outs eines Enzyms, wobei die Verringerung der Aktivität 90 oder mehr Prozent der Wildtyp-Aktivität ist, das eine der Reaktionen der β-Oxidation von Fettsäuren katalysiert ausgewählt aus der Gruppe umfassend FadE und FadL oder Varianten davon, wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen, als Teil der genetischen Ausstattung einer stoffwechselaktiven, prokaryotischen Zelle aufweisend eine rekombinante Alkanhydroxylase des alkB-Typs, zur Verbesserung der Abtrennung einer hydrophoben organischen Lösung von einem wässrigen Kulturmedium umfassend die stoffwechselaktive Zelle, wobei die organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst und

wobei "hydrophoben organischen Lösung" bedeutet, dass die organische Lösung einen P-Wert aufweist, dessen dekadischer Logarithmus größer 0 ist.

5. Prokaryotische Zelle, die eine ihrem Wildtyp gegenüber verringerte Aktivität eines Enzyms aufweist, das eine der Reaktionen der β-Oxidation von Fettsäuren katalysiert, weiter umfassend eine rekombinante Alkanhydroxylase des alkB-Typs,

wobei die Verringerung der Aktivität 90 oder mehr Prozent der Wildtyp-Aktivität ist,

wobei es sich bei dem Enzym, das eine der Reaktionen der β-Oxidation von Fettsäuren katalysiert, um ein Enzym aus der Gruppe FadE und FadL oder deren Varianten davon handelt,

wobei die Varianten eine andere Nukleinsäuresequenz, die mit Hinblick auf die entsprechende ursprüngliche Wildtyp-Nukleinsäuresequenz eine Identität von 70 oder mehr Prozent aufweist, darstellen und wobei die Varianten die gleiche enzymatische Aktivität des Wildtypmoleküls aufweisen.

6. Reaktionsmischung umfassend eine wässrige Lösung mit einer stoffwechselaktiven Zelle, gemäß Anspruch 5 und eine hydrophobe organische Lösung, wobei die hydrophobe organische Lösung wenigstens ein Lösungsmittel aus der Gruppe umfasst, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst und

wobei "hydrophoben organischen Lösung" bedeutet, dass die organische Lösung einen P-Wert aufweist, dessen dekadischer Logarithmus größer 0 ist.

7. Reaktionsmischung nach Anspruch 6, wobei die hydrophobe organische Lösung eine Fettsäure mit mehr als 12 Kohlenstoffatomen oder einen Ester davon umfasst, bevorzugt Ölsäure, Erukasäure oder Laurinsäuremethylester.

8. Verfahren nach einem der Ansprüche 1 oder 2, Verwendung nach einem der Ansprüche 3 oder 4 oder Reaktionsmischung nach einem der Ansprüche 6 oder 7, wobei es sich bei der stoffwechselaktiven Zelle um eine Zelle handelt, die eine Transaminase aufweist, bevorzugt darüber hinaus wenigstens ein Enzym aus der Gruppe umfassend Alkoholdehydrogenase, Alanindehydrogenase und Lactamhydrolase.

9. Verfahren nach einem der Ansprüche 1, 2 und 8, Verwendung nach einem der Ansprüche 3 oder 4 oder Reaktionsmischung nach einem der Ansprüche 6 oder 7, wobei die hydrophobe organische Lösung wenigstens 5, bevorzugt wenigstens 20 Prozent des Gesamtvolumens von hydrophober organischer Lösung und wässrigem Kulturmedium ausmacht.

10. Verfahren nach einem der Ansprüche 1, 2, 8 und 9, Verwendung nach einem der Ansprüche 3 oder 4 oder Reaktionsmischung nach einem der Ansprüche 6 oder 7, wobei der pH-Wert des wässrigen Kulturmediums zum Zeitpunkt des Kontaktierens zwischen 5 bis 9 liegt.

## Claims

1. Method comprising the steps of

    a) providing an aqueous culture medium comprising a metabolically active cell,
    b) contacting the aqueous culture medium with a hydrophobic organic solution,
    c) separating the hydrophobic organic solution from the aqueous culture medium,

wherein the cell exhibits a reduced activity, compared to the wild type of said cell, of at least one enzyme which catalyses one of the reactions of β-oxidation of fatty acids,
comprises a recombinant alkane hydroxylase of the alkB type,
wherein the cell is a prokaryotic cell,

wherein the reduction in activity is 90 per cent or more of the wild-type activity,
wherein the enzyme which catalyses one of the reactions of β-oxidation of fatty acids is an enzyme from the group consisting of FadE and FadL or their variants thereof,
wherein the variants are a different nucleic acid sequence which has, in respect of the corresponding original wild-type nucleic acid sequence, an identity of 70 per cent or more and wherein the variants exhibit the same enzymatic activity of the wild-type molecule,
wherein the organic solution comprises at least one solvent from the group which comprises substituted and unsubstituted alkanes, cycloalkanes, cycloalkenes, aryls, fatty acids, fatty acid esters, alcohols, heterocycloalkanes, heterocycloalkenes and heteroaryls that are liquid at room temperature, and
wherein "hydrophobic organic solution" means that the organic solution has a P value, the common logarithm of which is greater than 0.

2. Method according to Claim 1, wherein the organic solution comprises a fatty acid having more than 12 carbon atoms or an ester thereof, preferably oleic acid, erucic acid or methyl laurate.

3. Use of a metabolically active prokaryotic cell which exhibits a reduced activity, compared to the wild type of said cell, of an enzyme which catalyses one of the reactions of β-oxidation of fatty acids, selected from the group consisting of FadE and FadL and variants thereof,
comprising a recombinant alkane hydroxylase of the alkB type,
wherein the reduction in activity is 90 per cent or more of the wild-type activity,
wherein the variants are a different nucleic acid sequence which has, in respect of the corresponding original wild-type nucleic acid sequence, an identity of 70 per cent or more and wherein the variants exhibit the same enzymatic activity of the wild-type molecule,
for improving the separation of a hydrophobic organic solution from an aqueous culture medium comprising the metabolically active cell,
wherein the organic solution comprises at least one solvent from the group which comprises substituted and unsubstituted alkanes, cycloalkanes, cycloalkenes, aryls, fatty acids, fatty acid esters, alcohols, heterocycloalkanes, heterocycloalkenes and heteroaryls that are liquid at room temperature, and
wherein "hydrophobic organic solution" means that the organic solution has a P value, the common logarithm of which is greater than 0.

4. Use of a knockout of an enzyme, the reduction in activity being 90 per cent or more of the wild-type activity, which catalyses one of the reactions of β-oxidation of fatty acids, selected from the group comprising FadE and FadL or variants thereof, wherein the variants are a different nucleic acid sequence which has, in respect of the corresponding original wild-type nucleic acid sequence, an identity of 70 per cent or more and wherein the variants exhibit the same enzymatic activity of the wild-type molecule,
as part of the genetic makeup of a metabolically active prokaryotic cell comprising a recombinant alkane hydroxylase of the alkB type, for improving the separation of a hydrophobic organic solution from an aqueous culture medium comprising the metabolically active cell, wherein the organic solution comprises at least one solvent from the group which comprises substituted and unsubstituted alkanes, cycloalkanes, cycloalkenes, aryls, fatty acids, fatty acid esters, alcohols, heterocycloalkanes, heterocycloalkenes and heteroaryls that are liquid at room temperature, and wherein "hydrophobic organic solution" means that the organic solution has a P value, the common logarithm of which is greater than 0.

5. Prokaryotic cell which exhibits a reduced activity, compared to the wild type of said cell, of an enzyme which catalyses one of the reactions of β-oxidation of fatty acids, further comprising a recombinant alkane hydroxylase of the alkB type, wherein the reduction in activity is 90 per cent or more of the wild-type activity,
wherein the enzyme which catalyses one of the reactions of β-oxidation of fatty acids is an enzyme from the group consisting of FadE and FadL or their variants thereof,
wherein the variants are a different nucleic acid sequence which has, in respect of the corresponding original wild-type nucleic acid sequence, an identity of 70 per cent or more and wherein the variants exhibit the same enzymatic activity of the wild-type molecule.

6. Reaction mixture comprising an aqueous solution containing a metabolically active cell according to Claim 5 and a hydrophobic organic solution, wherein the hydrophobic organic solution comprises at least one solvent from the group which comprises substituted and unsubstituted alkanes, cycloalkanes, cycloalkenes, aryls, fatty acids, fatty acid esters, alcohols, heterocycloalkanes, heterocycloalkenes and heteroaryls that are liquid at room temperature, and

wherein "hydrophobic organic solution" means that the organic solution has a P value, the common logarithm of which is greater than 0.

7. Reaction mixture according to Claim 6, wherein the hydrophobic organic solution comprises a fatty acid having more than 12 carbon atoms or an ester thereof, preferably oleic acid, erucic acid or methyl laurate.

8. Method according to either one of Claims 1 and 2, use according to either one of Claims 3 and 4 or reaction mixture according to either one of Claims 6 and 7, wherein the metabolically active cell is a cell which comprises a transaminase, preferably additionally at least one enzyme from the group comprising alcohol dehydrogenase, alanine dehydrogenase and lactam hydrolase.

9. Method according to any one of Claims 1, 2 and 8, use according to either one of Claims 3 and 4 or reaction mixture according to either one of Claims 6 and 7, wherein the hydrophobic organic solution amounts to at least 5, preferably at least 20, per cent of the total volume of hydrophobic organic solution and aqueous culture medium.

10. Method according to any one of Claims 1, 2, 8 and 9, use according to either one of Claims 3 and 4 or reaction mixture according to either one of Claims 6 and 7, wherein the pH of the aqueous culture medium at the time of contacting is between 5 to 9.

**Revendications**

1. Procédé comprenant les étapes suivantes :

   a) la préparation d'un milieu de culture aqueux comprenant une cellule à activité métabolique,
   b) la mise en contact du milieu de culture aqueux avec une solution organique hydrophobe,
   c) la séparation de la solution organique hydrophobe du milieu de culture aqueux,

   la cellule présentant une activité réduite par rapport à son type sauvage d'au moins une enzyme qui catalyse une des réactions de β-oxydation d'acides gras,
   comprenant une hydroxylase d'alcanes recombinante de type alkB,
   la cellule étant une cellule procaryote,
   la réduction de l'activité étant de 90 pour cent ou plus de l'activité du type sauvage,
   l'enzyme qui catalyse une des réactions de β-oxydation d'acides gras étant une enzyme du groupe constitué par FadE et FadL ou leurs variantes de ceux-ci,
   les variantes consistant en une autre séquence d'acides nucléiques, qui présente une identité de 70 pour cent ou plus vis-à-vis de la séquence d'acides nucléiques de type sauvage initiale correspondante, et les variantes présentant la même activité enzymatique que la molécule de type sauvage,
   la solution organique comprenant au moins un solvant du groupe qui comprend les alcanes, cycloalcanes, cycloalcènes, aryles, acides gras, esters d'acides gras, alcools, hétérocycloalcanes, hétérocycloalcènes et hétéroaryles substitués et non substitués liquides à température ambiante, et
   « solution organique hydrophobe » signifiant que la solution organique présente une valeur P dont le logarithme décadien est supérieur à 0.

2. Procédé selon la revendication 1, dans lequel la solution organique comprend un acide gras contenant plus de 12 atomes de carbone ou un ester de celui-ci, de préférence l'acide oléique, l'acide érucique ou l'ester méthylique de l'acide laurique.

3. Utilisation d'une cellule procaryote à activité métabolique, qui présente une activité réduite par rapport à son type sauvage d'une enzyme qui catalyse une des réactions de β-oxydation d'acides gras, choisie dans le groupe constitué par FadE et FadL et leurs variantes, comprenant une hydroxylase d'alcanes recombinante de type alkB,
   la réduction de l'activité étant de 90 pour cent ou plus de l'activité du type sauvage,
   les variantes consistant en une autre séquence d'acides nucléiques, qui présente une identité de 70 pour cent ou plus vis-à-vis de la séquence d'acides nucléiques de type sauvage initiale correspondante, et les variantes présentant la même activité enzymatique que la molécule de type sauvage,
   pour l'amélioration de la séparation d'une solution organique hydrophobe d'un milieu de culture aqueux comprenant la cellule à activité métabolique,
   la solution organique comprenant au moins un solvant du groupe qui comprend les alcanes, cycloalcanes, cycloalcènes, aryles, acides gras, esters d'acides gras, alcools, hétérocycloalcanes, hétérocycloalcènes et hétéroaryles substitués et non substitués liquides à température ambiante, et

« solution organique hydrophobe » signifiant que la solution organique présente une valeur P dont le logarithme décadien est supérieur à 0.

4. Utilisation d'un knock-out d'une enzyme, la réduction de l'activité étant de 90 pour cent ou plus de l'activité du type sauvage, qui catalyse une des réactions de β-oxydation d'acides gras, choisie dans le groupe comprenant FadE et FadL ou leurs variantes, les variantes consistant en une autre séquence d'acides nucléiques, qui présente une identité de 70 pour cent ou plus vis-à-vis de la séquence d'acides nucléiques de type sauvage initiale correspondante, et les variantes présentant la même activité enzymatique que la molécule de type sauvage,

en tant que partie du matériel génétique d'une cellule procaryote à activité métabolique comprenant une hydroxylase d'alcanes recombinante de type alkB, pour l'amélioration de la séparation d'une solution organique hydrophobe d'un milieu de culture aqueux comprenant la cellule à activité métabolique, la solution organique comprenant au moins un solvant du groupe qui comprend les alcanes, cycloalcanes, cycloalcènes, aryles, acides gras, esters d'acides gras, alcools, hétérocycloalcanes, hétérocycloalcènes et hétéroaryles substitués et non substitués liquides à température ambiante, et

« solution organique hydrophobe » signifiant que la solution organique présente une valeur P dont le logarithme décadien est supérieur à 0.

5. Cellule procaryote, qui présente une activité réduite par rapport à son type sauvage d'une enzyme qui catalyse une des réactions de β-oxydation d'acides gras, comprenant en outre une hydroxylase d'alcanes recombinante de type alkB,

la réduction de l'activité étant de 90 pour cent ou plus de l'activité du type sauvage,

l'enzyme qui catalyse une des réactions de β-oxydation d'acides gras étant une enzyme du groupe constitué par FadE et FadL ou leurs variantes de ceux-ci,

les variantes consistant en une autre séquence d'acides nucléiques, qui présente une identité de 70 pour cent ou plus vis-à-vis de la séquence d'acides nucléiques de type sauvage initiale correspondante, et les variantes présentant la même activité enzymatique que la molécule de type sauvage.

6. Mélange réactionnel comprenant une solution aqueuse contenant une cellule à activité métabolique selon la revendication 5 et une solution organique hydrophobe, la solution organique hydrophobe comprenant au moins un solvant du groupe qui comprend les alcanes, cycloalcanes, cycloalcènes, aryles, acides gras, esters d'acides gras, alcools, hétérocycloalcanes, hétérocycloalcènes et hétéroaryles substitués et non substitués liquides à température ambiante, et

« solution organique hydrophobe » signifiant que la solution organique présente une valeur P dont le logarithme décadien est supérieur à 0.

7. Mélange réactionnel selon la revendication 6, dans lequel la solution organique hydrophobe comprend un acide gras contenant plus de 12 atomes de carbone ou un ester de celui-ci, de préférence l'acide oléique, l'acide érucique ou l'ester méthylique de l'acide laurique.

8. Procédé selon l'une quelconque des revendications 1 ou 2, utilisation selon l'une quelconque des revendications 3 ou 4 ou mélange réactionnel selon l'une quelconque des revendications 6 ou 7, dans lequel ou dans laquelle la cellule à activité métabolique est une cellule qui comprend une transaminase, de préférence en outre au moins une enzyme du groupe comprenant les déshydrogénases d'alcools, les déshydrogénases d'alanines et les hydrolases de lactames.

9. Procédé selon l'une quelconque des revendications 1, 2 et 8, utilisation selon l'une quelconque des revendications 3 ou 4 ou mélange réactionnel selon l'une quelconque des revendications 6 ou 7, dans lequel ou dans laquelle la solution organique hydrophobe représente au moins 5, de préférence au moins 20 pour cent du volume total de la solution organique hydrophobe et du milieu de culture aqueux.

10. Procédé selon l'une quelconque des revendications 1, 2, 8 et 9, utilisation selon l'une quelconque des revendications 3 ou 4 ou mélange réactionnel selon l'une quelconque des revendications 6 ou 7, dans lequel ou dans laquelle le pH du milieu de culture aqueux au moment de la mise en contact est compris entre 5 à 9.

Fig. 1

Fig. 2

Fig. 3

**Fig. 4**

Fig. 5a

Fig. 5b

Fig. 5c

Fig. 5d

**Fig. 6a**

**Fig. 6b**

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009077461 A

- EP 11154707 A

**In der Beschreibung aufgeführte Nicht-Patentliteratur**

- Octanol-Water Partition Coefficients: Fundamentals and Physical Chemistry. **J. SANGSTER.** Wiley Series in Solution Chemistry. John Wiley & Sons, 1997, vol. 2
- **ARTHUR LESK.** Introduction to bioinformatics. 2008
- The DIG System Users Guide for Filter Hybridization. Handbuch. Firma Boehringer Mannheim GmbH, 1993
- **LIEBL et al.** *International Journal of Systematic Bacteriology,* 1991, vol. 41, 255-260
- Hybaid Hybridisation Guide. Hybaid Limited, 1996
- The DIG System User's Guide for Filter Hybridisation. Boehringer Mannheim, 1995
- Octanol-Water Partition Coefficients: Fundamentals and Physical Chemistry. **J SANGSTER.** Wiley Series in Solution Chemistry. John Wiley & Sons, 1997, vol. 2
- **F M AUSUBEL.** Current Protocols in Molecular Biology. John Wiley & Sons, Inc, 1995
- **A M LESK.** Introduction to Bioinformatics. 2008
- **Y FUJITA ; H MATSUOKA ; K HIROOKA.** *Mol. Microbiology,* 2007, vol. 66 (4), 829-839
- **P N BLACK.** *J. Bacteriol.,* 1991, vol. 173, 435-442
- **P N BLACK ; C C DIRUSSO ; A K METZGER ; T L HEIMERT.** *J. Biol. Chem.,* 1992, vol. 267, 25513-25520
- **J. W CAMPBELL ; J E CRONAN.** *J. Bacteriol.,* vol. 184, 3759-3764

- **A CORNISH-BOWDEN.** Fundamentals of Enzym Kinetics. Portland Press Limited, 1995
- **S LOBO ; G FLOROVA ; KA REYNOLDS.** *Biochemistry,* 2001, vol. 40 (39), 11955-64
- **X YU ; T LIU ; F ZHU ; C KHOSLA.** elektronische Veröffentlichung vor Drucklegung. *PNAS,* 2011
- **K KAMEDA ; W D NUNN.** *J. Biol. Chem.,* 1981, vol. 256, 5702-5707
- **HI MARRAKCHI ; W E DEWOLF ; C QUINN ; J WEST ; B J POLIZZI ; C Y SO et al.** *Biochem. J.,* 2003, vol. 370, 1055-1062
- **SAMBROOK ; FRITSCH ; MANIATIS.** Molecular Cloning: A Laboratory Manual. Cold Spring Harbor Laboratory Press, 1989
- **FUCHS ; SCHLEGEL.** Allgemeine Mikrobiologie. Georg Thieme Verlag, 2007
- **B A MOFFATT ; F W STUDIER.** *J. Mol. Biol.,* 1986, vol. 189, 113-130
- **A H ROSENBERG ; B N LADE ; D CHUI ; S LIN ; J J DUNN ; F W STUDIER.** *Gene,* 1987, vol. 56, 125-135
- **F W STUDIER ; A H ROSENBERG ; J J DUNN ; J W DUBENDORFF.** *Meth. Enzymol.,* 1990, vol. 185, 60-89
- **C GRANT ; J M WOODLEY ; F BAGANZ.** *Enzyme and Microbial Technology,* 2011, 480-486
- **D J KOCH ; M M CHEN ; J B VAN BEILEN ; F H ARNOLD.** *Appl. and Env. Microbiology,* 2009, 337-344
- **T TUSCHL.** *ChemBioChem,* 2001, vol. 2, 239-145